# EUROPEAN PATENT SPECIFICATION

(11) **EP 2 077 754 B1**
(45) Date of publication and mention of the grant of the patent: **23.03.2011**
(21) Application number: 07826697.0
(22) Date of filing: 09.10.2007
(51) Int. Cl.: A61B 5/103, A61F 2/68

(54) **SWITCHABLE JOINT CONSTRAINT SYSTEM**
SCHALTBARES VERBINDUNGSEINSCHRÄNKUNGSSYSTEM
SYSTÈME DE CONTRAINTE D'ARTICULATION COMMUTABLE

(30) Priority: 13.10.2006 EP 06122243
(43) Date of publication of application: 15.07.2009
(73) Proprietor: Koninklijke Philips Electronics N.V., 5621 BA Eindhoven (NL); Philips Intellectual Property & Standards GmbH, 20099 Hamburg (DE)
(72) Inventor: LANFERMANN, Gerd, NL-5656 AE Eindhoven (NL); WILLMANN, Richard, D., NL-5656 AE Eindhoven (NL); TE VRUGT, Juergen, NL-5656 AE Eindhoven (NL); BONGERS, Edwin, G., J., M., NL-5656 AE Eindhoven (NL)
(74) Representative: Van Velzen, Maaike Mathilde
(86) International application number: PCT/IB2007/054112
(87) International publication number: WO 2008/044207

(56) References cited:
- DE-A1- 19 859 931
- US-A1- 2002 146 672
- US-A1- 2006 135 883
- US-B1- 6 344 062
- LIEPERT J ET AL: "Motor cortex plasticity during constraint-induced movement therapy in stroke patients" NEUROSCIENCE LETTERS, LIMERICK, IE, vol. 250, no. 1, 26 June 1998 (1998-06-26), pages 5-8, XP004863825 ISSN: 0304-3940
- PAGE ET AL: "Back From the Brink: Electromyography-Triggered Stimulation Combined With Modified Constraint-Induced Movement Therapy in Chronic Stroke" ARCHIVES OF PHYSICAL MEDICINE AND REHABILITATION, W.B. SAUNDERS, vol. 87, no. 1, January 2006 (2006-01), pages 27-31, XP005235731 ISSN: 0003-9993

## Description

### BACKGROUND OF THE INVENTION

The present invention concerns a switchable joint constraint system.

Half sided paralysis (hemiplegy) is one of the most common symptoms of neurological disorders. For example, about 1.8 million people in Western countries suffer from stroke every year. Their rehabilitation should be aimed at restoring the functional use of the affected limb, especially in the activities of daily living. The use of the disabled limb is a promising approach to achieve rehabilitation success. However, people prefer not to use the disabled limb. It is common that they simply forget about it. This leads to a further worsening of the condition of the disabled limb. In order to enforce the use of the impaired limb, doctors may put the healthy limb in a plaster cast or restrain it with orthoses. While this "constraint induced therapy" (CIT) is a rather rigorous method, positive results may be obtained. However, completely immobilizing a limb keeps patients from performing those activities that require two limbs such as carrying a tray. Furthermore, patients may exhaust the motor capabilities of the impaired limb.

US 2006/0135883 A1 and US 6,344,062 disclose control systems and methods for processing a time series of signals associated with the movement of a device associated with a limb. The time series of motion signals is filtered, such as through an autoregressive filter, and compared to stored data sets representing a limb-motion event and/or phase. In certain examples, a plurality of accelerometers generate the time series of motion signals, based on at least acceleration measurements in three orthogonal directions and/or planes. The acceleration measurements may relate to the movement of an artificial limb, such as a prosthetic or orthotic device. Upon determining an event and/or phase of limb motion, the control system may trigger an actuator to appropriately adjust one or more prosthetic or orthotic joints.

However, this system are directed towards actively enabling the movement of a joint, for example via an actuator, instead of blocking it. Furthermore, this systems are directed towards the facilitation of limb movements, such as walking, without taking into account further situations.

### SUMMARY OF THE INVENTION

A joint constraint system suitable for use in the constraint induced therapy which allows for varying situations where both limbs need to be used while also constraining the movement of the joint when the affected limb should be trained would still be desirable.

To better address one or more of these concerns, a switchable joint constraint system is presented which comprises:
- at least one motion sensor,
- a joint constraint which is switchable between a mobile and an immobile state,
- a processing unit which receives signals from the at least one motion sensor and which issues switching commands to the joint constraint,
- a database in communication with the processing unit,
- wherein the database stores data from the at least one motion sensor resolved into individual spatial descriptors and time, and
- the database furthermore comprises comparison data resolved into individual spatial descriptors and time,
- which comparison data is assigned to at least one predefined situation, and
- the processing unit compares the data from the at least one motion sensor to the data assigned to the at least one predefined situation, and
- the processing unit issues switching commands to the joint constraint, based on whether the data from the at least one motion sensor corresponds to comparison data.

### BRIEF DESCRIPTION OF THE DRAWINGS

Fig. 1 is a block diagram of the components of the system according to the present invention showing the interactions between them.
Fig. 2 shows a patient wearing sensors and a joint constraint.

### DETAILED DESCRIPTION OF THE INVENTION

Before the invention is described in detail, it is to be understood that this invention is not limited to the particular component parts of the devices described or process steps of the methods described, as such devices and methods may vary. It is also to be understood that the terminology used herein is for purposes of describing particular embodiments only, and is not intended to be limiting. It must be noted that, as used in the specification and the appended claims, the singular forms "a," "an" and "the" include singular and/or plural referents unless the context clearly dictates otherwise.

The individual spatial descriptors in a system according to the present invention can be any parameters that are appropriate to describe the spatial position of e.g. a sensor or the orientation of a limb. For example, such individual spatial descriptors may be Cartesian coordinates (x, y, z), Euler angles or quaternions.

The at least one motion sensor in a system according to the present invention may be an inertial sensor. The class of inertial sensors comprises, for example, accelerometers, gyroscopes and magnetometers. Accelerometers may be sensitive to the earth's gravitational field, whereas gyroscopes may be sensitive to the rate of turn.

For example, the acceleration sensor may detect acceleration along the x-, y- and z-axis. It is also possible that the acceleration sensor may detect angular acceleration, such as angular acceleration in three substantially orthogonal planes. It is also possible that the motion sensor is a combined sensor which combines, for example, a three-dimensional accelerometer, two two-dimensional gyroscopes and three one-dimensional magnetometers. The sensor or sensors may be located at various positions on the body of the person using the joint constraint system according to the present invention, such as on the arms, legs and/or torso. The sensors may record the movement of the immobilized limb as well as the movement of the impaired limb.

The joint constraint which is switchable between a mobile and an immobile state serves to block the motion of a limb by immobilizing a joint. It can be placed removably onto the joint in order, for example, to facilitate hygienical operations, such as washing of the person, using the system according to the present invention. Switching between a mobile state, where the joint may be used, and an immobile state, where the joint cannot be moved and therefore a limb is immobilized, may be undertaken via electrical or electronic commands.

The processing unit may comprise signal filters for processing a multitude of simultaneous sensor signals. It may incorporate a microprocessor as a central processing unit. The signals that the processing unit receives from the at least one motion sensor may be raw signals or pre-processed signals. In any case, the processing unit resolves the data from the at least one motion sensor into individual spatial descriptors and time. Furthermore, the processing unit is capable of issuing commands to the joint constraint which cause the joint constraint to adopt a mobile or immobile state.

The database is in communication with the processing unit, that is the processing unit is able to read and write data into the database.

The database has already stored, that is it already comprises, comparison data. This comparison data has the same structure as the data that the processing unit writes into the database. The comparison data is assigned to at least one predefined situation. This means that a certain variation of the individual spatial descriptors provided via a sensor or several sensors within a given time interval is brought into connection with a certain situation. For example, a variation of the coordinates of the sensors may indicate that the person wearing the system according to the present invention is falling. Another situation could be that the person is resting.

The processing unit then compares the data from the at least one motion sensor to the data in the database which is assigned to at least one situation. This can be undertaken by comparing the present data that has been written into the database to the stored data. As a result of this comparison the processing unit issues switching commands to the joint constraint, based upon whether a certain situation is met.

For example, the processing unit may determine that the person wearing the system is inactive and therefore issue a command to switch the joint constraint into a mobile state. Another example would be if the processing unit determines that the person wearing the system is falling. In this case, the joint constraint would also be switched to a mobile state so that the falling person may use the now mobile limb in order to protect himself from injury. A further example would be that the system determines that the person is walking. A constrained arm would be released in order to support a well-balanced gait.

According to a preferred embodiment of the present invention, the system further comprises a user interface for accessing data from the database. With this user interface, a medical professional may see the amount of activity that the immobilized limb and the affected limb have experienced. The activity may be calculated by integrating three-dimensional data from the sensors over the period of time. Therefore, the medical professional is able to more accurately assess the situation regarding the affected limb. Furthermore, an activity threshold for the patient may be established. This describes the maximum amount of activity that the affected limb may tolerate or the minimum amount of activity that the immobilized limb should still experience. If the upper activity threshold for the affected limb or the lower activity threshold for the impaired limb is reached, the joint constraint is switched to a mobile state so that the working limb may support the affected limb. This prevents overexertion of the affected limb and weakening of the impaired limb.

According to a further preferred embodiment of the present invention, the system further comprises a display unit displaying the status of the system with respect to at least the movement or position of at least one motion sensor. This provides the possibility of giving the patient feedback. For example, the patient may be informed whether he has used the affected limb long enough in order to complete his training program.

According to a further preferred embodiment of the present invention, the signals from at least one motion sensor to the processing unit and/or the commands from the processing unit to the switchable joint constraint are transmitted wirelessly. The wireless transmission may, for example, be undertaken via a variety of commercially available wireless transmission technologies like Bluetooth, infrared, WLAN (wireless local area network) and the like. It is also possible to use a proprietary protocol. By eliminating the need for cables the convenience of use for the patient is increased as well as the safety, because the patient cannot stumble over these cables any more.

According to a further preferred embodiment of the present invention, the joint constraint is switched between a mobile and an immobile state by applying an electrical field to an electrorheological fluid or by applying a magnetic field to a magnetorheological fluid. In rheological fluids, particles align when exposed to the appropriate energy field. When this alignment occurs, the ability of the fluid to flow, or shear, is substantially reduced. One fluid type is responsive to a voltage field (electrorheological fluids) and the other type is responsive to a magnetic field (magnetorheological fluids). In other words, these "smart fluids" solidify in the presence of an electrical or magnetic field and re-liquefy when this field is removed.

The rheological fluid may be enclosed in a soft garment and be worn around the joint that is to be immobilized in a cuff-like fashion. The use of these fluids is beneficial in that no bulky or moving parts are needed to switch from a mobile to an immobile state, thus allowing for lightweight and unobtrusive joint constraints. Furthermore, the garments allow for a good fit around the patient's joint, thus reducing the danger of chafing.

According to a further preferred embodiment of the present invention, the joint constraint is switched between a mobile and an immobile state by an inflatable cuff. Inflatable cuffs stiffen when air is inflated into chambers around the joint. This provides for a low-cost and low-maintenance joint constraint.

According to a further preferred embodiment of the present invention, the joint constraint is switched between a mobile and an immobile state by locking the joints of an orthosis. Orthoses provide good control over the joint movement as they are tightly attached to the limb. The joints may be either fully locked, fully opened or open only to allow a certain degree of movement. In the use of orthoses it is beneficial that the joints may be locked at any arbitrary angle. When the joint only allows for a certain degree of freedom, the immobilized limb may be immobilized enough to force the patient to use the impaired limb, while at the same time involuntary stiffening of the immobilized limb due to prolonged non-use is prevented.

According to a further preferred embodiment of the present invention, the system further comprises a release unit which switches the joint constraint to a mobile state after a manual input. This is beneficial when the patient wishes to perform actions which are not recognized by the system and thus do not lead to the switching of the joint constraint to a mobile state. Examples of such situations include everyday actions like opening a jar with a screw-on lid when a hand or elbow joint is immobilized. The release unit may take the form of a switch or may be activated via voice control. The release unit may furthermore be configured to switch the joint constraint to a mobile state for a limited time in order to avoid cheating by the patient. Furthermore, the use of the release unit may be recorded in the database.

According to a further preferred embodiment of the present invention, the system further comprises sensors selected from the group comprising electromyographical sensors, skin perspiration sensors, pulse sensors, blood pressure sensors and/or blood oxygen level sensors, and the sensors further provide input to the processing unit. These sensors may provide further information regarding the status of the patient. They may especially relate to the stress status of the patient. For example, an electromyographical sensor gives information about the fatigue of a muscle. This is useful in assessing whether the impaired limb is overexerted and should be supported by the other limb. The same applies to skin perspiration sensors, which give information about the electrical conductivity of the skin surface, which changes according to the level of perspiration caused by stress and fatigue. Pulse sensors, blood pressure sensors and/or blood oxygen level sensors may be integrated into one sensor system which can be worn, for instance, on the tip of a finger or can be attached to an ear lobe with a clip. They are useful in providing data related to the circulatory system of the patient. The input from all the aforementioned sensors may be used by the processing unit to more precisely determine whether a predefined situation is encountered or not.

A further embodiment concerns a process for handling sensor signals in a system according to the present invention, comprising the steps of:
a) calibrating the at least one motion sensor by bringing the at least one motion sensor into a predetermined position and registering the output signal from the at least one motion sensor in this position
b) gathering motion sensor output signals
c) assigning the motion sensor output signals to a position and a movement of the respective at least one motion sensor
d) storing the position and movement signals in a database
e) comparing the position and movement signals which have been stored within a predetermined timeframe to predetermined position and movement data in the database, which predetermined position and movement data corresponds to predefined situations
f) deciding whether a predefined situation is met and switching the joint constraint to an appropriate state for the situation, as defined in a decision table.

In step a) in this process the user may be asked to bring himself into a certain posture. For example, he may be asked to stand upright and let his arms hang freely along the sides of his body. Then the system is able to correlate the sensor output signals with a certain position of the limbs and to subsequently calculate the current position of the limbs.

Step b) describes the continuous data gathering by the motion sensors. In step c) the motion sensor output signals are assigned to a position and a movement of a sensor. This can be undertaken either in the sensor itself or within the processing unit of the system. In other words, the sensor signals are resolved into individual spatial descriptors and a time dimension. This data which represents the position and movement of at least one sensor is then stored in a database, as mentioned in step d).

Step e) comprises the comparison of the position and movement signals, which have been stored in the database, to previously stored data, which is assigned to a certain situation. This can be undertaken by the processing unit of the system. If the variation of the spatial coordinates of one or more sensors over a given amount of time matches the variation of the previously stored data, the system issues a command to switch the joint constraint to an appropriate status. When to switch and which status is to be reached is defined in a decision table within the database, as mentioned in step f). For example, the system may decide that the person is falling and then switch the joint constraint to a mobile state so that the person may use both limbs in order to prevent injury.

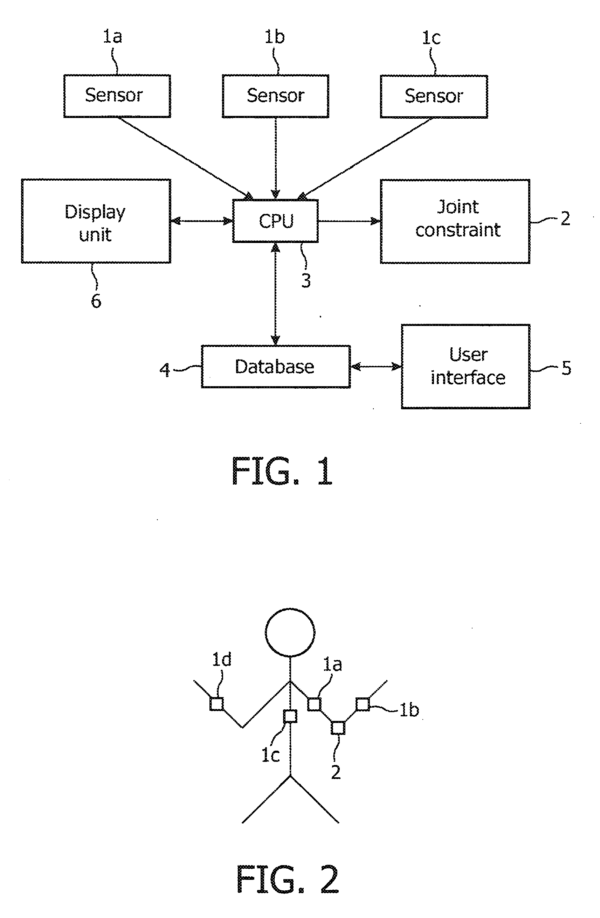

Fig.1 illustrates a block diagram of the components of the system according to the present invention, showing the interactions between them. The individual components are three motion sensors (1a, 1b, 1c), a display unit (6), a central processing unit (CPU, 3), a joint constraint (2), a database (4) and a user interface (5). The sensors (1a, 1b, 1c) provide input to the CPU (3), which processes the input into data with spatial and time coordinates. This data is stored in the database (4) together with comparison data. The CPU (3) then continually compares the sensor data to the comparison data. Based upon this comparison, that is, when a predefined situation is met, the CPU (3) orders the joint constraint (2) to switch into a mobile state or back into an immobile state. The stored data in the database (4) may be viewed by a user such as a physiotherapist or a physician via a user interface (5). They can review the activity of the limbs with sensors on them and set threshold conditions when the joint constraint is to be activated or not. Furthermore, a display unit (6) is in communication with the CPU (3). It is capable of displaying the status of the system with respect to at least the movement or position of at least one sensor (1). For example, the display unit (6) may show if an impaired limb or immobilized limb is being moved correctly within a target corridor defined by speed of movement or extent of movement. The display unit (6) may also give audio signals to indicate, for example, whether a limb is being used enough or not.

Fig. 2 shows a patient wearing motion sensors (1a, 1b, 1c, 1d) and a joint constraint (2). The healthy limb and thus the limb to be immobilized in the constraint induced therapy bears two motion sensors (1a, 1b) and a joint constraint (2) around the elbow joint. A further motion sensor (1c) is located around the torso of the patient. A fourth motion sensor (1d) is located on the impaired limb. The sensors (1a, 1b, 1c, 1d) and the joint constraint (2) are in wireless contact with the rest of the system.

The particular combinations of elements and features in the above detailed embodiments are exemplary only; the interchanging and substitution of these teachings with other teachings in this application and the patents/applications incorporated by reference are also expressly contemplated. As those skilled in the art will recognize, variations, modifications, and other implementations of what is described herein can occur to those of ordinary skill in the art without departing from the spirit and the scope of the invention as claimed. Accordingly, the foregoing description is by way of example only and is not intended as limiting. The invention's scope is defined in the following claims and the equivalents thereto. Furthermore, reference signs used in the description and claims do not limit the scope of the invention as claimed.

## Claims

1. A switchable joint constraint system, comprising:
- at least one motion sensor (1),
- a joint constraint (2) which is switchable between a mobile and an immobile state,
- a processing unit (3) which receives signals from the at least one motion sensor (1) and which issues switching commands to the joint constraint (2),
- a database (4) in communication with the processing unit (3),
- wherein the database (4) stores data from the at least one motion sensor (1) resolved into individual spatial descriptors and time, and
- the database (4) furthermore comprises comparison data resolved into individual spatial descriptors and time,
- which comparison data is assigned to at least one predefined situation, and
- the processing unit (3) compares the data from the at least one motion sensor to the data assigned to the at least one predefined situation, and
- the processing unit (3) issues switching commands to the joint constraint (2), based on whether the data from the at least one motion sensor corresponds to comparison data.

2. System according to claim 1, further comprising a user interface (5) for accessing data from the database (4).

3. System according to claim 1, further comprising a display unit (6) displaying the status of the system with respect to at least the movement or position of at least one motion sensor (1).

4. System according to claim 1, wherein the signals from at least one motion sensor (1) to the processing unit (3) and/or the commands from the processing unit (3) to the switchable joint constraint (2) are transmitted wirelessly.

5. System according to claim 1, wherein the joint constraint (2) is switched between a mobile and an immobile state by applying an electrical field to an electrorheological fluid or by applying a magnetic field to a magnetorheological fluid.

6. System according to claim 1, wherein the joint constraint (2) is switched between a mobile and an immobile state by an inflatable cuff.

7. System according to claim 1, wherein the joint constraint (2) is switched between a mobile and an immobile state by locking the joints of an orthosis.

8. System according to claim 1, further comprising a release unit which switches the joint constraint (2) to a mobile state after a manual input.

9. System according to claim 1, further comprising sensors selected from the group comprising electromyographical sensors, skin perspiration sensors, pulse sensors, blood pressure sensors and/or blood oxygen level sensors, and wherein the sensors further provide input to the processing unit.

## Patentansprüche

1. Umschaltbares Gelenkeinschränkungssystem, das Folgendes umfasst:
- mindestens einen Bewegungssensor (1),
- eine Gelenkeinschränkung (2), die zwischen einem beweglichen und einem unbeweglichen Zustand umschaltbar ist,
- eine Verarbeitungseinheit (3), die Signale von dem mindestens einen Bewegungssensor (1) empfängt und die Umschaltbefehle an die Gelenkeinschränkung (2) ausgibt,
- eine Datenbank (4) in Kommunikation mit der Verarbeitungseinheit (3),
- wobei die Datenbank (4) Daten von dem mindestens einen Bewegungssensor (1) speichert, die in einzelne räumliche Deskriptoren und Zeit aufgelöst sind, und
- die Datenbank (4) weiterhin Vergleichsdaten umfasst, die in einzelne räumliche Deskriptoren und Zeit aufgelöst sind,
- wobei die Vergleichsdaten mindestens einer vordefinierten Situation zugeordnet sind, und
- die Verarbeitungseinheit (3) die Daten von dem mindestens einen Bewegungssensor mit den der mindestens einen vordefinierten Situation zugeordneten Daten vergleicht, und
- die Verarbeitungseinheit (3) Umschaltbefehle an die Gelenkeinschränkung (2) darauf basierend ausgibt, ob die Daten von dem mindestens einen Bewegungssensor den Vergleichsdaten entsprechen.

2. System nach Anspruch 1, weiterhin mit einer Benutzerschnittstelle (5) zum Zugreifen auf die Daten aus der Datenbank (4).

3. System nach Anspruch 1, weiterhin mit einer Anzeigeeinheit (6), welche den Status des Systems in Bezug auf mindestens die Bewegung oder die Position des mindestens einen Bewegungssensors (1) anzeigt.

4. System nach Anspruch 1, wobei die Signale von dem mindestens einen Bewegungssensor (1) an die Verarbeitungseinheit (3) und/oder die Befehle von der Verarbeitungseinheit (3) an die umschaltbare Gelenkeinschränkung (2) drahtlos übertragen werden.

5. System nach Anspruch 1, wobei die Gelenkeinschränkung (2) zwischen einem beweglichen und einem unbeweglichen Zustand umgeschaltet wird, indem ein elektrisches Feld an ein elektrorheologisches Fluid angelegt wird oder indem ein Magnetfeld an ein magnetorheologisches Fluid angelegt wird.

6. System nach Anspruch 1, wobei die Gelenkeinschränkung (2) durch eine aufblasbare Manschette zwischen einem beweglichen und einem unbeweglichen Zustand umgeschaltet wird.

7. System nach Anspruch 1, wobei die Gelenkeinschränkung (2) durch Arretieren der Gelenke einer Orthese zwischen einem beweglichen und einem unbeweglichen Zustand umgeschaltet wird.

8. System nach Anspruch 1, weiterhin mit einer Freigabeeinheit, die die Gelenkeinschränkung (2) nach einer manuellen Eingabe auf einen beweglichen Zustand umschaltet.

9. System nach Anspruch 1, weiterhin mit Sensoren, die aus der Gruppe bestehend aus elektromyographischen Sensoren, Hauttranspirationssensoren, Impulssensoren, Blutdrucksensoren und/oder Blutsauerstoffgehalt-Sensoren ausgewählt werden, und wobei die Sensoren weiterhin die Eingabe für die Verarbeitungseinheit liefern.

## Revendications

1. Système de contrainte d'articulation commutable, comprenant :
- au moins un capteur de mouvement (1),
- une contrainte d'articulation (2) qui est commutable entre un état mobile et un état immobile,
- une unité de traitement (3) qui reçoit des signaux à partir de l'au moins un capteur de mouvement (1) et qui produit des commandes de commutation à la contrainte d'articulation (2),
- une base de données (4) en communication avec l'unité de traitement (3),
- dans lequel la base de données (4) stocke les données provenant de l'au moins un capteur de mouvement (1) résolues en descripteurs spatiaux individuels et temps, et
- la base de données (4) comprend en outre des données de comparaison résolues en descripteurs spatiaux individuels et temps,
- lesquelles données de comparaison sont affectées à au moins une situation prédéfinie, et
- l'unité de traitement (3) compare les données provenant de l'au moins un capteur de mouvement aux données affectées à l'au moins une situation prédéfinie, et
- l'unité de traitement (3) produit des commandes de commutation à la contrainte d'articulation (2), selon si les données provenant de l'au moins un capteur de mouvement correspondent aux données de comparaison.

2. Système selon la revendication 1, comprenant en outre une interface utilisateur (5) pour accéder à des données de la base de données (4).

3. Système selon la revendication 1, comprenant en outre une unité d'affichage (6) affichant le statut du système par rapport à au moins le mouvement ou la position d'au moins un capteur de mouvement (1).

4. Système selon la revendication 1, dans lequel les signaux allant d'au moins un capteur de mouvement (1) à l'unité de traitement (3) et/ou les commandes allant de l'unité de traitement (3) vers la contrainte d'articulation commutable (2) sont transmis sans fil.

5. Système selon la revendication 1, dans lequel la contrainte d'articulation (2) est commutée entre un état mobile et un état immobile en appliquant un champ électrique à un fluide électro-rhéologique ou en appliquant un champ magnétique à un fluide magnéto-rhéologique.

6. Système selon la revendication 1, dans lequel la contrainte d'articulation (2) est commutée entre un état mobile et un état immobile par une manchette gonflable.

7. Système selon la revendication 1, dans lequel la contrainte d'articulation (2) est commutée entre un état mobile et un état immobile en verrouillant les articulations d'une orthèse.

8. Système selon la revendication 1, comprenant en outre une unité de libération qui commute la contrainte d'articulation (2) à un état mobile après une entrée manuelle.

9. Système selon la revendication 1, comprenant en outre des capteurs choisis parmi le groupe comprenant des capteurs électromyographiques, des capteurs de transpiration de la peau, des capteurs d'impulsions, des capteurs de pression sanguine et/ou des capteurs de niveau d'oxygène sanguin, et dans lequel les capteurs fournissent en outre une entrée à l'unité de traitement.
